# EUROPÄISCHE PATENTANMELDUNG

(11) **EP 2 042 160 A1**
(43) Veröffentlichungstag der Anmeldung: **01.04.2009**
(21) Anmeldenummer: 07116502.1
(22) Anmeldetag: 14.09.2007
(51) Int. Cl.: A61K 9/00

(54) **Mittel für die Behandlung von Mund und Rachen in Form von Tabletten**

(71) Anmelder: BASF SE, 67056 Ludwigshafen (DE)
(72) Erfinder: Die Erfindernennung liegt noch nicht vor

(57) **Zusammenfassung**

Mittel für die Anwendung in Mund und Rachen, welche neben mindestens einem Wirkstoff als Matrixkomponente eine Mischung aus
a)60 - 98 Gew.-% mindestens eines Zuckers oder Zuckeralkohols oder Mischungen davon,
b) 1 - 25 Gew.-% eines Sprengmittels,
c) 1 - 15 Gew.-% von wasserunlöslichen Polymeren,
d) 0 - 15 Gew.-% wasserlöslichen Polymeren, und
e) 0 - 15 Gew.-% weiterer pharmazeutisch üblichen Hilfsstoffen,

wobei die Summe der Komponenten a) bis e) 100 Gew.-% beträgt, enthalten.

## Beschreibung

Die vorliegende Erfindung betrifft Formulierungen in Form von Agglomeraten für die Herstellung von Tabletten, enthaltend Zucker oder Zuckeralkohole, quervernetztes Polyvinylpyrrolidon und wasserunlösliche Polymere, zur Herstellung von Darreichungsformen für die Anwendung in Mund und Rachen.

Im Mund schnell zerfallende und/oder sich schnell auflösende Tabletten gewinnen für die orale Applikation von Arzneistoffen immer größere Bedeutung. Solche Tabletten müssen innerhalb kurzer Zeit, am besten innerhalb von 30 Sekunden in der Mundhöhle zerfallen, angenehm schmecken und dürfen kein sandiges Gefühl hinterlassen. Ferner sollen sie einfach herstellbar sein, wobei die Direkttablettierung erhebliche Vorteile gegenüber der Feuchtgranulation bietet, und eine hohe mechanische Festigkeit besitzen, damit sie Verpackungsprozeduren, Transporte und auch das Herausdrücken aus Verpackungen unbeschadet überstehen. Aber auch Kau- und Lutschtabletten, die sich ebenfalls im Mund auflösen, gewinnen Zunehmend an Bedeutung

Gerade auch für die Anwendung zur Pflege von Mund auch Rachen, wie beispielsweise zu Verhinderung von Mundgeruch oder Karies, zur Zahnbleichung oder zur Desinfektion des Rachens sind solche Darreichungsformen von Interesse. In Form von Tabletten lassen sich Wirkstoffe für die genannten Anwendungen im Vergleich zu flüssigen oder pastösen Formulierungen genauer zu dosieren.

Schnell zerfallende Tabletten bestehen häufig aus Zucker und Zuckeralkoholen, Brausesystemen, mikrokristalliner Cellulose und anderen nicht wasserlöslichen Füllstoffen Calciumhydrogenphosphat, Cellulose-Derivaten, Maisstärke, oder Polypeptiden. Weiterhin kommen wasserlösliche Polymere, übliche Sprengmittel (quervernetztes PVP, Na- und Ca-Salz der quervernetzten Carboxymethylcellulose, Na-Salz der Carboxymethylstärke, niedrigsubstituierte Hydroxypropylcellulose L-HPC) und im wesentlichen anorganische wasserunlösliche Bestandteile (Kieselsäuren, Silikate, anorganische Pigmente) zum Einsatz. Weiterhin können die Tabletten auch Tenside enthalten.

In der WO 2003/051338 ist eine direkttablettierbare und gut verpressbare Hilfsstoffformulierung, welche Mannit und Sorbit enthält, beschrieben. Zunächst wird durch Lösen von Mannit und Sorbit in Wasser und anschließender Sprühtrocknung (gewöhnliche Sprühtrocknung und SBD-Verfahren) eine Hilfsstoffvormischung hergestellt. Dieser coprozessierten Mischung kann zusätzlich Mannit zugesetzt werden. Tabletten, welche zusätzlich Sprengmittel, Trennmittel, Pigment und einen Wirkstoff enthalten, sollen innerhalb von 60 Sekunden in der Mundhöhle zerfallen.

In der US 2002/0071864 A1 wird eine Tablette beschrieben, welche innerhalb von 60 Sekunden in der Mundhöhle zerfällt, und hauptsächlich aus einer physikalischen Mischung von sprühgetrocknetem Mannit und einem grobkörnigen quervernetztem Polyvinylpyrrolidon sowie einer begrenzten Auswahl an Wirkstoffen formuliert ist. Diese Tabletten besitzen eine Bruchfestigkeit von ca. 40N und erzeugen ein unangenehmes, sandiges Mundgefühl.

Gemäß der US 6,696,085 B2 soll ein Methacrylsäure-Copolymer Typ C als Zerfallsmittel eingesetzt werden. Das Methacrylsäure-Copolymer Typ C ist ein magensaftresistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

EP 0839526 A2 beschreibt eine pharmazeutische Darreichungsform bestehend aus einem Wirkstoff, Erythrit, kristalliner Cellulose und einem Sprengmittel. Weiterhin wird Mannit eingearbeitet und als Sprengmittel quervernetztes Polyvinylpyrrolidon verwendet, sodass eine physikalische Mischung entsteht. Die Tabletten sollen innerhalb von 60 Sekunden in der Mundhöhle zerfallen.

In der Anmeldung JP 2004-265216 wird eine im Mund innerhalb von 60 Sekunden zerfallende Tablette, bestehend aus einem Wirkstoff, einem wasserlöslichen Polyvinylalkohol-Polyethylenglykol-Copolymer, Zucker/Zuckeralkohol (Mannit) und Sprengmittel, beschrieben.

Desodorierende und Karies hemmende Tabletten sind beispielsweise aus der US 2,894,876, der US 3,238,844 oder der US 3,556,811, bekannt. In der US 4,041,149 werden Formulierungen in Form von Mundwasser, Zahnpasta oder Zahncreme beschrieben, die als Füllstoff einen Zuckerlakohol enthalten können. Beschrieben werden auch Polyvinylacetat enthaltende Kaugummis.

Tabletten für die Mundpflege auf Basis von Zuckeralkoholen, enthaltend eine Wirkstoffkombination von Zimksalzen und lononverbindungen, sind aus der DE 37 06 142 A bekannt.

Die Matrixkomponenten auf Basis von Zuckeralkoholen, Sprengmitteln und unlöslichen Polymeren sind allgemein für pharmazeutische Anwendungen aus der WO 2007/071581 bekannt.

Aufgabe der vorliegenden Erfindung war es, verbesserte Mittel für die Anwendung in Mund und Rachen zu finden, die nicht nur ein angenehmes Mundgefühl hinterlassen und mechanisch sehr stabil sind, sondern auch eine einfache Anwendung und Dosierung ermöglichen und auch ohne zusätzliche Anwendung von Flüssigkeit verabreicht werden können..

Demgemäß wurden Mittel für die Anwendung in Mund und Rachen gefunden, welche neben mindestens einem Wirkstoff für die Pflege von Mund und Rachen als Matrixkomponente eine Mischung aus
a) 60 - 98 Gew.-% mindestens eines Zuckers oder Zuckeralkohols oder Mischungen davon,
b) 1 - 25 Gew.-% eines Sprengmittels,
c) 1 - 15 Gew.-% von wasserunlöslichen Polymeren,
d) 0 - 15 Gew.-% wasserlöslichen Polymeren, und
e) 0 - 15 Gew.-% weiterer Hilfsstoffe,
, wobei die Summe der Komponenten a) bis e) 100 Gew.-% beträgt, enthalten.

Weiterhin wurden Darreichungsformen, insbesondere im Mund schnell zerfallende Tabletten, enthaltend solche Zubereitungen, gefunden. Die schnell zerfallendenTabletten zerfallen im Mund oder in wässrigem Milieu innerhalb von 40 Sekunden, bevorzugt innerhalb von 30 Sekunden, besonders bevorzugt innerhalb von 20 Sekunden. Ebenso wurden Lutsch- und Kautabletten erhältlich aus solchen Mitteln gefunden. Ebenso wurde die Verwendung solcher Mittel in Form von Sachets gefunden.

Wirkstoffe für die Pflege von Mund und Rachen bedeuten erfindungsgemäß Wirkstoffe für die Mund- und Zahnhygiene, zur Behandlung von Mundgeruch, Karies, Zahnbelägen, Zahnverfärbungen und Feindesinfektion der Mund- und Rachenschleimhaut.

Die Zubereitungen enthalten als Komponente a) 60 bis 98 Gew.-%, bevorzugt 70 bis 95 Gew.-%, besonders bevorzugt 75 bis 93 Gew.-% eines Zuckers, Zuckeralkohols oder Mischungen davon. Als Zucker oder Zuckeralkohole eignen sich Trehalose, Mannit, Erythrit, Isomalt, Maltit, Lactit, Xylit, Sorbit. Die Zucker- oder Zuckeralkoholkomponenten sind bevorzugt feinteilig, mit mittleren Teilchengrößen von 5 bis 100 µm. Gewünschtenfalls können die Teilchengrößen durch Mahlen eingestellt werden. Bevorzugt werden Mannit, Erythrit oder Mischungen davon eingesetzt.

Als Komponente b) werden Sprengmittel in Mengen von 1 bis 25 Gew.-%, bevorzugt, 2 bis 15 Gew.-%, besonders bevorzugt 3 bis 10 Gew.-%, eingesetzt. Solche Sprengmittel sind wasserunlöslich, aber nicht filmbildend. Als Sprengmittel eignet sich quervernetztes Polyvinylpyrrolidon (Crospovidon), Croscarmellose, eine quervernetzte Carboxymethyllellulose, wobei als Croscarmellose erfindungsgemäß auch deren Natrium- und Calciumsalze gemeint sind. Weiterhin eignet sich Natriumcarboxymethylstärke. Ebenso eignet sich L-Hydroxypropylcellulose, bevorzugt mit 5 bis 16 % Hydroxypropoxygruppen, wie in USP/NF 2005 beschrieben. Bevorzugt wird Crospovidon verwendet.

Als Komponente c) werden wasserunlösliche Polymere eingesetzt in Mengen von 1 bis 15 Gew.-%, bevorzugt 1 bis 10 Gew.-%, eingesetzt. Dabei handelt es sich um Polymere , . Bevorzugt sind Polymere, die im pH-Bereich von 1 bis 14 unlöslich sind, also eine bei jedem pH-Wert pH-unabhängige Wasserunlöslichkeit aufweisen. Weiterhin eignen sich aber auch Polymere, die bei jedem pH-Wert im pH-Bereich von 6 bis 14 wasserunlöslich sind.

Die Polymere sollen filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen.

Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrylat-Methylmethacrylat-Dimethylaminoethylmethacrylat-Terpolymere Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E. Als bevorzugte Komponente c) kommt Polyvinylacetat zum Einsatz. Dieses kann als wässrige Dispersion mit Feststoffgehalten von 10 bis 45 Gew.-% eingesetzt werden. Bevorzugt ist zudem Polyvinylacetat mit einem Molekulargewicht zwischen 100.000 und 1.000.000 Dalton besonders bevorzugt zwischen 200.000 und 800.000 Dalton.

Weiterhin können die Formulierungen als Komponenten d) wasserlösliche Polymere in Mengen von 0 bis 15 Gew.-% enthalten. Geeignete wasserlösliche Polymere sind beispielsweise Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere, Hydroxypropylmethylcellulose, Hydroxypropylcellulose, Hydroxyethylcellulose, Carragenane, Pektine, Xanthane, Alginate.

Gewünschtenfalls können durch Zusatz von pharmazeutisch üblichen Hilfsstoffen (Komponenten e)) in Mengen von 0 bis 15 Gew.-%, beispielsweise wie Säuerungsmitteln, Puffersubstanzen, Süßstoffen, Aromen, Geschmacksverstärkern und Farbstoffen Geschmack und Aussehen der aus den Formulierungen erhaltenen Tabletten weiter verbessert werden.

### Folgende Stoffe sind hierbei besonders geeignet:

Als Säuerungsmitttel eignen sich Citronensäure, Weinsäure, Ascorbinsäure, Natriumdihydrogenphosphat, oder ... .

Als Süßstoffe eignen sich: Cyclamat, Saccharin-Na, Aspartam, Neohesperidin

Als Aromen eignen sich beispielsweise Fruchtaromen, Vanillearoma, Kakaoaroma, Glutamat,

Als Farbstoffe eignen sich: Riboflavin, Curcumin, Betacarotin, wasserlösliche Farbstoffe wie sie zur Färbung von Lebensmitteln Verwednung finden sowie feinteilige Farblacke.

Durch Zusatz von Verdickungsmitteln wie hochmolekularen Polysacchariden kann das Mundgefühl durch Erhöhung der Weichheit und des Volumengefühls zusätzlich verbessert werden.

Weiterhin können als Komponenten e) auch Tenside zugegeben werden. Als Tenside eignen sich beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine. Weiterhin eignet sich Natriumstearylfumarat.

Weiterhin können zur weiteren Verbesserung des Zerfalls auch feinteilige Pigmente zugegeben werden, weil sie die inneren Grenzflächen erhöhen und somit Wasser schneller in die Tablette eindringen kann. Diese Pigmente wie Eisenoxide, Titandioxid, kolloidale oder gefällte Kieselsäure, Calciumcarbonate, Calciumphosphate müssen natürlich sehr feinteilig sein, ansonsten entsteht wiederum ein körniger Geschmack.

Die Mischung aus den Komponenten a) bis e) kommt vorzugsweise in Form von Agglomeraten zur Anwendung.

Die Herstellung der Agglomerate kann durch Aufbau-Agglomeration in Mischern, Wirbelschichtgeräten oder Sprühtürmen erfolgen. Dabei werden feste Ausgangsmaterialien und Granulierflüssigkeit zunächst miteinander vermischt und das feuchte Mischgut anschließend getrocknet. Gemäß der vorliegenden Erfindung wird als Granulierflüssigkeit eine wässrige Dispersion der Komponente c), des wasserunlöslichen Polymers, eingesetzt.

Bei der Agglomeration in der Wirbelschicht wird eine wässrige Dispersion des wasserunlöslichen Polymers auf eine wirbelnde Mischung aus Zucker oder Zuckeralkohol und Sprengmittel gesprüht, wodurch die feinen Teilchen agglomerieren. Die Zulufttemperaturen betragen 30 bis 100°C, die Ablufttemperaturen 20 bis 70°C.

Bei der Herstellung in Sprühtürmen wird vorzugsweise die sogenannte FSD- oder SBD-Technologie (FSD: Fluidized spray drying; SBD: Spray bed drying) eingesetzt. Hierbei wird eine Lösung des Zuckers oder Zuckeralkohols in Wasser zunächst sprühgetrocknet, im unteren Teil des Sprühtrockners oder in einem angeschlossenen Wirbelbett erfolgt die Zugabe von Sprengmittel und die Eindüsung einer wässrigen Dispersion des wasserunlöslichen Polymeren, wodurch die Teilchen agglomerieren. Feine Teilchen können ferner nochmals vor die Sprühdüse der Zucker oder Zuckeralkohollösung geblasen werden und zusätzlich agglomeriert werden. Es ist im Sprühturm, FSD oder SBD auch eine Prozeßführung ausgehend von der kristallinen Form des Zuckers oder Zuckeralkohols möglich. Dabei wird der kristalline Zucker oder Zuckeralkohol am Kopf des Sprühturms oder in den Feingut-Recyclingstrom zugegeben. Durch das Versprühen einer wässrigen Dispersion des wasserunlöslichen Polymeren wird dieser kristalline Feststoff im Turm agglomeriert.

Sprengmittel Für den Agglomerationsprozess kann es sich als günstig erweisen, einen mehrstufigen Sprühprozess zu fahren. Zu Anfang wird die Sprührate niedrig gehalten, um ein Überfeuchten der Produktvorlage und damit deren Verkleben zu verhindern. Mit zunehmender Prozessdauer kann die Sprührate erhöht und damit die Agglomerationstendenz erhöht werden. Es ist ebenfalls möglich die Zuluftmenge und/oder -temperatur in entsprechender Weise während des Prozesses anzupassen. Besonders während der Trocknungsphase ist es vorteilhaft, die Zuluftmenge zu reduzieren und damit einem Abrieb der Agglomerate durch eine hohe mechanische Belastung vorzubeugen.

Als weitere Anpassungsparameter für die Agglomeratgröße kann die Feinheit des Sprühtröpfchens der Bindemittellösung bzw. -dispersion (einstellbar über den Zerstäubungsgasdruck), die Düsengeometrie und Abstand der Düse zum Produktbett angesehen werden. Je feiner und einheitlicher gesprüht wird, desto feiner und einheitlicher resultieren die Agglomerate. Je weiter die Düse vom Produktbett entfernt ist, desto schlechter ist das Agglomerationsverhalten.

Weiterhin können die Agglomerate auch in einem Mischer durch eine kontinuierlich geführte Mischaggregation erfolgen. Eine solche kontinuierlich geführte Form der Mischaggregation ist die sogenannte "Schugi-Granulation". Dabei werden in einem kontinuierlich arbeitenden vertikal angeordneten Hochgeschwindigkeitsmischer feste Ausgangsmaterialien und die das wasserunlösliche Polymer enthaltende Granulierflüssigkeit intensiv miteinander vermischt (siehe auch M. Bohnet, "Mechanische Verfahrenstechnik", Wiley VCH Verlag, Weinheim 2004, S. 198 ff.)

Gemäß einer besonderen Ausführungsform wird das Sprengmittel in der wässrigen Dispersion des wasserunlöslichen Polymers suspendiert.
Die so erhaltenen Agglomerate weisen eine mittlere Teilchengröße von 70 - 600 µm, bevorzugt 120 - 500 µm und besonders bevorzugt von 140 - 400 µm auf.
Das wasserunlösliche, filmbildende Polymer dient dabei als Agglomerationsmittel, um die feinen Zucker oder Zuckeralkoholkristalle und die Teilchen des Sprengmittels zu agglomerieren.

Die erfindungsgemäßen Formulierungen können vorteilhaft für die Herstellung von Mitteln zur Behandlung von Mund und Rachen verwendet werden.

Die Mittel zur Behandlung von Mund und Rachen enthalten zusätzlich zu der als Matrixbildner wirkenden Mischung Wirkstoffe speziell für die Pflege und Behandlung von Mund und Rachen ("Oral Care"). Darunter ist erfindungsgemäß Mund- und Zahnhygiere, Bekämpfung von Halitosis (Mundgeruch), Karies, Zahnbelägen, Zahnverfärbungen und der Feindesinfektion zur Bekämpfung von mikrobiell verursachten entzündlichen Veränderungen der Mund- und Rachenschleimhaut zu verstehen.

### Geeignete Wirkstoffe können sein:

Komplexe von Polyvinylpyrrolidon-lod und/oder Polyvyinlpyrrolidon-Wasserstoffperoxid, wobei es sich bei dem Polyvinylpyrrolidon sowohl um wasserlösliches Polyvinylpyrrolidon als auch um wasserunlösliches quervernetztes Polyvinylpyrrolidon handeln kann. Bevorzugt ist wasserlösliches Polyvinylpyrrolidon. Die Herstellung solcher, auch kommerziell verfügbarer, Komplexe ist dem Fachmann an sich bekannt.

Zinksalze wie die Fluoride, Chloride, Jodide, Gluconate , Phenolsulfonate,

### Kupfersalze wie die Gluconate

Anorganische Fluorsalze des Natriums, Kaliums, Ammoniums, Calciums, Kupfer(I), Bariums, Zinks, , weiterhin die Fluorsilikate oder Fluorzirkonate von Natrium, Kalium und Ammonium, Mono- oder Difluorphosphate von Natrium oder Aluminium, fluoriertes Natrium Calciumpyrophosphat.

Weiterhin eigenen sich als Wirkstoffe die folgenden organischen Verbindungen:
Phenolische Verbindungen wie:
Eucalyptol, Thymol, Methylsalicylat, Menthol, Chlorthymol, Phenol, halogenierte und andere Derivate des Phenols, wie Methoxy-4-(2-Propenyl)phenol (Eugenol), Alkyl-p-chlorphenole, Alkyl-p-Bromphenole, Alkyl-o-Bromphenole und dergleichen.

### Resorcin und seine Derivate

### Bisphenolische Verbindungen

Halogenierte Diphenylether wie beispielsweise 2,4,4'-Trichlor-2'-hydroxydiphenlyether (Triclosan)

Halogenierte Salicylanilide wie beispielsweise 3,5-dibrom-3'-trifluor-methylsalicylanilid (Fluorophen)

Benzoesäureester wie beispielsweise Methyl-p-hydroxybenzoeester

### Halogenierte Carbanilide

Solche geeigneten organischen Verbindungen sind beispielsweise in der DE695 23 513 T2, auf die hiermit ausdrücklich Bezug genommen wird, aufgelistet.

Cetylpyridiniumchlorid , quarternäre Ammoniumverbindungen wie z.B. das Didecyldimethylammoniumchlorid oder das Benzalkoniumchlorid

Die Menge der eingesetzten aktiven Bestandteile in einer Formulierung variiert wirkstoffabhängig zwischen 0,01 % und 25%, meistens bevorzugt in einem Bereich von 0,1% bis 15%.

Zur Herstellung der erfindungsgemäßen Mittel werden Wirkstoffe und Matrixbildner und vorzugsweise Schmiermittel miteinander vermischt, wobei auch ein zusätzlicher Granulationsschritt ausgeführt werden kann.

Für die Herstellung von Tabletten können die üblichen Verfahren verwendet werden, wobei die Direkttablettierung und die Walzenkompaktierung besondere Vorteile bieten. Aufgrund der besonderen Eigenschaften der erfindungsgemäßen Formulierungen werden in der Regel nur Wirkstoff, erfindungsgemäße Formulierung und ein Schmiermittel benötigt. Die Tablettenrezeptur ist somit sehr einfach, sehr reproduzierbar und das Verfahren leicht zu validieren.

Es ist auch möglich Zwei- oder Mehrschichttabletten herzustellen, wobei in den unterschiedlichen Schichten unterschiedliche Wirkstoffe eingearbeitet werden können.

Überraschenderweise wurde gefunden, dass ein wasserunlösliches filmbildendes Polymer den Zerfall von Tabletten erheblich beschleunigt. Dies ist umso überraschender, da solche Polymere in der Regel für die Herstellung von retardierten Arzneiformen eingesetzt werden, die innerhalb mehrerer Stunden nicht zerfallen. Die Zerfallszeiten unter Verwendung von Polyvinylacetat als wasserunlöslichem Polymer sind erheblich kürzer als bei wasserlöslichen Polymeren.

Ferner besitzen die erfindungsgemäßen Mittel außergewöhnlich gute Fließfähigkeiten und Verpressbarkeiten, die zu mechanisch sehr stabilen Tabletten führen. Die Bruchfestigkeit der mit Hilfe der erfindungsgemäßen pharmazeutischen Formulierungen erzeugten Tabletten beträgt > 50 N. Häufig liegen die Bruchfestigkeiten oberhalb von 80 N, selbst unter Verwendung schwer verpressbarer Wirkstoffe. Die Friabilitäten betragen < 0,2 %. Beschädigungen beim üblichen Tablettenhandling treten somit nicht auf.

Über den Pressdruck kann auch die Auflösungsgeschwindigketi gesteuert werden. Will man schnell zerfallende Tabletten herstellen, wird man niedrigere bis mittlere Pressdrücke im Bereich von 3 bis 10 kN wählen. Will man Lutsch- oder Kautabletten herstellen empfehlen sich höhere Pressdrücke im Bereich von 8 bis15kN.

Die erfindungsgemäßen Formulierungen sind damit bei Lagerung sehr stabil und behalten ihr ansprechendes Aussehen.

### Beispiele

Beispiele A - F zeigen den zerfallsfördernden Effekt von Polyvinylacetat als wasserunlösliches Polymer gegenüber wasserlöslichen Polymeren.

Zunächst wurden Agglomerate in der Wirbelschicht hergestellt: Zucker/Zuckeralkohol und quervernetztes PVP wurden vorgelegt und mit wässrigen Bindemittellösungen/- dispersionen, die in den Wirbelschichtgranulator ( Firma Glatt,GPCG 3.1) mittels Topspray-Verfahren eingesprüht wurden, agglomeriert. Erythrit wurde aufgrund seiner groben Körnung zunächst zu einem feinen Pulver zerkleinert.

Die Herstellung erfolgte durch einen zweistufigen Agglomerationsprozess, wobei zunächst eine geringere Sprührate gewählt wurde und dann die Sprührate erhöht wurde.

Folgende Herstellungsbedingungen wurden in einem zweistufigen Agglomerationsprozess angewendet:

| | |
|---|---|
| Ansatzgröße: | 0,6 kg |
| Konzentration der Bindemittellösung/-dispersion: | 10 Gwe.-% |
| Zulufttemperatur: | 55 °C |
| Ablufttemperatur zu Beginn: | 35 °C |
| Ablufttemperatur nach Umstellen der Sprührate: | 25 °C |
| Sprührate zu Beginn: | 7,5 g/min |
| Sprührate nach Umstellen: | 20 g/min |

Als wässrige Bindemitteldispersion wurde eine kommerziell erhältliche Polyvinylacetatdispersion (Kollicoat® SR30 D, Fa. BASF AG) verwendet. Als L-Hydroxypropylcellulose wurde eine Type mit einem Hydroxypropoxy-Gehalt von 11 % verwendet.

**Tabelle 1: Rezepturzusammensetzung in Gew.%**

| | A | B | C | D | E | F |
|---|---|---|---|---|---|---|
| Mannitol (d_{0,5}:36µm] | 90 | 90 | 90 | 44 | 76 | 90 |
| Erythritol (d_{0,5}:44µm] | | | | 44 | | |
| Sorbitol (d_{0,5}:47µm] | | | | | 11 | |
| Crospovidon | | | | | | 5 |
| Croscarmellose-Na | 5 | | | | | |
| Croscarmellose-Ca | | | | 8 | | |
| quervernetzte Natriumcarboxymethylstärke | | 5 | | | | |
| L-Hydroxypropylcellulose | | | 5 | | | |
| Kollicoat SR30D (Feststoff) | 5 | 5 | 5 | 4 | 6 | 5 |

Die so hergestellten Agglomerate wurden mit wasserlöslichen oder wasserunlöslichen Schmiermitteln (z.B. Polyethylenglyol hoher Molmasse oder Mg-Stearat) in einem Turbulamischer gemischt. Anschließend wurden diese Mischungen auf einer voll instrumentierten Rundläufertablettenpresse (Korsch PH 100/6) bei einer Umdrehungsgeschwindigkeit von 30 Upm tablettiert. Die Rundläufertablettenpresse war mit 6 Stempeln (10 mm, biplan, facettiert) ausgestattet. Das Tablettengewicht wurde auf 300 mg eingestellt. Zunächst erfolgte eine Tablettierung bei einer Presskraft von 18 kN (die Tabletten wurden je nach Verpressbarkeit des Pulvers unterschiedlich hart), anschließend wurde die Presskraft jeweils so eingestellt, dass die Bruchfestigkeit der Tabletten 60 N betrug.

### Beispiel 1

| Formulierung mit PVP-lod | | | | | |
|---|---|---|---|---|---|
| (2 mg verfügbares lod pro 150mg Tablette) | | | | | |
| | Einsatzstoff | Zusammensetzung | | Gewichtsanteile in Tabletten | |
| 1 | Formulierung F | g | 551,5 | mg | 110,3 |
| 2 | PVP-lod 30/06 M10 | g | 100,0 | mg | 20,0 |
| 3 | Eucalyptol, kristallin | g | 17,5 | mg | 3,5 |
| 4 | Menthol, kristallin | g | 20,0 | mg | 4,0 |
| 5 | Aspartame | g | 7,5 | mg | 1,5 |
| 6 | Aerosil® 200 | g | 12,0 | | 2,4 |
| 7 | Polyethylenglycol 6000, | g | 41,5 | mg | 8,3 |
| | Feinpulver | | | | |
| | Total | g | 750,0 | mg | 150,0 |

Die Komponenten 1 bis 7 werden durch ein Sieb von 0,8mm passiert. Zunächst werden die Komponenten 1-6 in einem V-Blender über eine Zeitdauer von 20 Minuten gemischt. Anschließend wird die Komponente 7 zugesetzt und nochmals für 5 Minuten gemischt.

Die homogene Tablettenmischung wird zu Tabletten verpresst. Es werden konvexe Tabletten mit einem Durchmesser von 8mm und mit einem Gewicht von 150mg hergestellt. Der Pressdruck wird auf 8kN eingestellt.

### Beispiel 2

| Zahnfleisch- und Rachendesinfektionstablette: Farblose Formulierung mit in-situ gebildetem Crospovidon-lod | | | | | |
|---|---|---|---|---|---|
| (5mg verfügbares lod pro 100mg Tablette) | | | | | |
| | Einsatzstoff | Zusammensetzung | | Gewichtsanteile in Tab letten | |
| 1 | Formulierung F | g | 227,5 | mg | 75,0 |
| 2 | Kollidon® CL-SF | g | 10,0 | mg | 3,3 |
| 3 | NaJ | g | 24,0 | mg | 8,0 |
| 4 | NaJO3 | g | 4,0 | mg | 1,5 |
| 5 | Zitronensäure, wasserfrei, Feinpulver | g | 20,0 | mg | 6,6 |
| 6 | Eucalyptol, kristallin | g | 5,0 | mg | 1,5 |
| 7 | Menthol, kristallin | g | 5,0 | mg | 1,6 |
| 8 | Aspartame | g | 2,0 | mg | 0,6 |
| 9 | Aerosil® 200 | g | 4,0 | mg | 1,3 |
| 10 | Magnesiumstearat | g | 2,0 | mg | 0,6 |
| | Summe | g | 303,5 | mg | 100,0 |

Die Komponenten 1 bis 9 werden durch ein Sieb mit der Maschenweite 800µm gegeben und danach in einem Turbula-Mischer für 15 Minuten gemischt. Anschließend wird das ebenfalls durch das o.g. Sieb passierte Magnesiumstearat als Schmiermittel zugesetzt und die Mischung für weitere 5 Minuten gemischt.

Die Mischung wird dann auf einem Rundläufer zu 6 mm Tabletten in konvexer Form und 100mg Tablettengewicht verpresst. Pro Tablette können in dem saurem Medium 5mg an verfügbarem lod freigesetzt und mit Crospovidone komplexiert werden.

### Beispiel 3

| Formulierung mit Chlorophyllin-Kupferkomplex, Na-Salz | | | | | |
|---|---|---|---|---|---|
| (10mg Chlorophyllin pro 125mg Tablette) | | | | | |
| | Einsatzstoff | Zusammensetzung | | Gewichtsanteile in Tabletten | |
| 1 | Formulierung F | g | 840,0 | mg | 105,0 |
| 2 | Chlorophyllin, Kupferkomplex, Na-Salz | g | 80,0 | mg | 10,0 |
| 3 | Pfefferminz Aroma, microverkapselt | g | 32,0 | mg | 4,0 |
| 4 | Polyethylenglycol 4000, Feinpulver | g | 48,0 | mg | 6,0 |
| | Summe | g | 1000,0 | mg | 125,0 |

Die Komponenten 1 bis 4 werden durch ein Sieb von 0,8mm passiert. Zunächst werden 300g der Komponente 1 mit den Komponenten 2 und-3 in einem Turbulamischer über eine Zeitdauer von 12 Minuten gemischt. Zu dieser Vormischung wird die restliche Menge der Komponente 1 gegeben und die Mischung final 10 Minuten gemischt. Anschließend wird die Komponente 4 zugesetzt und nochmals für 5 Minuten gemischt.

Die homogene Tablettenmischung wird zu Tabletten verpresst. Es werden konvexe Tabletten mit einem Durchmesser von 8mm und mit einem Gewicht von 125mg hergestellt. Der Pressdruck wird auf 6kN eingestellt.

Der schnell zerfallende Hilfsstoff wird durch Agglomeration in der Wirbelschicht von Mannit (90 Gew.-%) und quervernetztem PVP (5 Gew.-%) mit Polyvinylacetat (5 Gew.-%) hergestellt. Das so hergestellte Direkttablettierungsmittel wurde mit Wirkstoff und 0,5 bis 1,0 Gew.-% Schmiermittel (Mg-Stearat) abgemischt und anschließend auf einer Rundläufertablettenpresse (Korsch PH 100/6) zu Tabletten mit 60 N Bruchfestigkeit verpresst.

### Beispiel 4

| Formulierung mit PVP-Wasserstoffperoxid Komplex zur Desinfektion ds Mund und Rachenraumes sowie zur Zahnbleichung | | | | | |
|---|---|---|---|---|---|
| (2,5 mg Wasserstoffperoxid in 250mg Tablette) | | | | | |
| | Einstzstoff | Zusammensetzung | | Gewichtsanteile in Tabletten | |
| 1 | Formulierung F | g | 454,0 | mg | 227,0 |
| 2 | PVP-Wasserstoffperoxid | g | 25,0 | mg | 12,5 |
| | komplex, 20% | | | | |
| 3 | Lemon & Lime Aroma | g | 8,0 | mg | 4,0 |
| 4 | Aspartame | g | 4,0 | mg | 2,0 |
| 5 | Aerosil® 200 | g | 5,0 | mg | 2,5 |
| 6 | Magnesiumstearat | g | 4,0 | mg | 2,0 |
| | Total | g | 500,0 | mg | 250,0 |

Die Komponenten 1 bis 5 werden durch ein Sieb von 0,8mm passiert. Zunächst werden die Komponenten 1-6 in einem V-Blender über eine Zeitdauer von 15 Minuten gemischt. Anschließend wird die Komponente 6 zugesetzt und nochmals für 5 Minuten gemischt.

Die homogene Tablettenmischung wird zu Tabletten verpresst. Es werden konvexe Tabletten mit einem Durchmesser von 12mm und mit einem Gewicht von 250mg hergestellt. Der Pressdruck wird auf 7kN eingestellt.

### Beispiel 5

| Formulierung mit Kamillenextrakt | | | | | |
|---|---|---|---|---|---|
| (1,5% Kamillenextrakt in 150mg Tablette) | | | | | |
| | Einstzstoff | Zusammensetzung | | Gewichtsanteile in Tabletten | |
| 1 | Formulierung F | g | 1090,0 | mg | 109,0 |
| 2 | Kamilllenextrakt, Pulverform | g | 280,0 | mg | 28,0 |
| 3 | Menthol, kristallin | g | 40,0 | mg | 4,0 |
| 4 | Aerosil® 200 | g | 10,0 | mg | 1,0 |
| 5 | Polyethylenglycol 4000, Feinpul | g | 80,0 | mg | 8,0 |
| | ver | | | | |
| | Total | g | 1500,0 | mg | 150,0 |

Die Komponenten 1 bis 5 werden durch ein Sieb von 0,8mm passiert. Die Komponenten 1 bis 4 werden 15 Minuten in einem Turbulamischer gemischt. Anschließend wird die Komponente 5 zugesetzt und nochmals für 5 Minuten gemischt.

Die homogene Tablettenmischung wird zu Tabletten verpresst. Es werden konvexe Tabletten mit einem Durchmesser von 8mm und mit einem Gewicht von 125mg hergestellt. Der Pressdruck wird auf 8kN eingestellt.

### Beispiel 6

| Formulierung mit Zinkgluconat zur Halitosis Beseitigung | | | | | |
|---|---|---|---|---|---|
| (2,5 mg Zink in 100mg Tablette) | | | | | |
| | Einstzstoff | Zusammensetzung | | Gewichtsanteile in Tabletten | |
| 1 | Formulierung F | g | 70,5 | mg | 70,5 |
| 2 | Zinkglukonat | g | 17,5 | mg | 17,5 |
| 3 | Grapefruit Aroma | g | 5,0 | mg | 5,0 |
| 4 | Aerosil® 200 | g | 1,0 | mg | 1,0 |
| 5 | Polyethylenglycol 6000, | g | 6,0 | mg | 6,0 |
| | Feinpulver | | | | |
| | Total | g | 100,0 | mg | 100,0 |

Die Komponenten 1 bis 5 werden durch ein Sieb von 0,8mm passiert. Zunächst werden die Komponenten 1-4 in einem Turbulamischer über eine Zeitdauer von 15 Minuten gemischt. Anschließend wird die Komponente 5 zugesetzt und nochmals für 5 Minuten gemischt.

Die homogene Tablettenmischung wird zu Tabletten verpresst. Es werden konvexe Tabletten mit einem Durchmesser von 6mm und mit einem Gewicht von 100mg hergestellt. Der Pressdruck wird auf 5kN eingestellt.

## Patentansprüche

1. Mittel für die Anwendung in Mund und Rachen, welche neben mindestens einem Wirkstoff als Matrixkomponente eine Mischung aus
a)60 - 98 Gew.-% mindestens eines Zuckers oder Zuckeralkohols oder Mischungen davon,
b) 1 - 25 Gew.-% eines Sprengmittels,
c) 1 - 15 Gew.-% von wasserunlöslichen Polymeren,
d) 0 - 15 Gew.-% wasserlöslichen Polymeren, und
e) 0 - 15 Gew.-% weiterer pharmazeutisch üblichen Hilfsstoffen,
wobei die Summe der Komponenten a) bis e) 100 Gew.-% beträgt, enthalten.

2. Mittel nach Anspruch 1, enthaltend als Zuckeralkohol a) Mannitol.

3. Mittel nach Anspruch 1 oder 2, enthaltend als Komponente b) ein Polymer ausgewählt aus der gruppe bestehend aus quervernetztem Polyvinylpyrrolidon (Crospovidon), Croscarmellose, Natrium- und Calciumsalze der Croscarmellose, Natriumcarboxymethylstärke L-Hydroxypropylcellulose.

4. Mittel nach einem der Ansprüche 1 bis 3, enthaltend als Komponente b) Crospovidon.

5. Mittel nach einem der Ansprüche 1 bis 4, enthaltend als Komponente c) Polyvinylacetat, Ethylcellulose, Copolymere der (Meth)acrylsäure oder Mischungen davon.

6. Mittel nach einem der Ansprüche 1 bis 5, enthaltend als Komponente d) Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere oder Mischungen davon.

7. Mittel nach einem der Ansprüche 1 bis 6, enthaltend als Komponente e) pharmazeutisch übliche Hilfsstoffen wie Säuerungsmittel, Puffersubstanzen, Süßstoffe, Aromen, Geschmacksverstärker und Farbstoffe.

8. Mittel nach einem der Ansprüche 1 bis 7, enthaltend als Wirkstoff ein Mittel zur Bekämpfung von Mundgeruch, Karies, Zahnverfärbungen, Zahnbelägen und mikrobiell verursachten Erkrankungen der Mund- und Rachenschleimhaut.

9. Mittel nach einem der Ansprüche 1 bis 8, enthaltend als Wirkstoff einen Komplex von Polyvinylpyrrolidon-lod oder Polyvinylpyrroldion-Wasserstoffperoxid oder Mischungen davon, wobei das Polyvinylpyrrolidon wasserlöslich oder wasserunlöslich quervernetzt sein kann.

10. Mittel nach einem der Ansprüche 1 bis 9, enthaltend als Wirkstoff eine phenolische Verbindung.

11. Mittel nach einem der Ansprüche 1 bis 10, enthaltend als Wirkstoff ein Zink- oder Kupfersalz.

12. Mittel nach einem der Ansprüche 1 bis 11, enthaltend als Wirkstoff ein anorganisches Fluoridsalz.

13. Mittel nach einem der Ansprüche 1 bis 12, in Form einer durch Komprimierung erhaltenen Tablette oder Pastille.

14. Mittel nach einem der Ansprüche 1 bis 13, in Form von im Mund schnell zerfallenden Tabletten.

15. Mittel nach einem der Ansprüche 1 bis 14, in Form einer Lutsch- oder Kautablette.

16. Mittel nach einem der Ansprüche 1 bis 11, in Form eines Granulats.

17. Mittel nach einem der Ansprüche 1 bis 16, enthaltend einen oder mehrere Wirkstoffe in Mengen 0.01 bis 25 Gew.-%, bezogen auf das Gesamtgewicht des Mittels.

18. Verwendung von Mitteln nach einem der Ansprüche 1 bis 17, zur Pflege von Mund und Rachen.

19. Verwendung nach Anspruch 18, zur Behandlung von Mundgeruch, Karies, Zahnbelag, Zahnverfärbungen und mikrobiell verursachten Erkrankungen der Mund- und Rachenschleimhaut.
